# EUROPÄISCHE PATENTSCHRIFT

(11) **EP 2 654 823 B1**
(45) Veröffentlichungstag und Bekanntmachung des Hinweises auf die Patenterteilung: **08.02.2017**
(21) Anmeldenummer: 11808169.4
(22) Anmeldetag: 20.12.2011
(51) Int. Cl.: A61M 1/00, A61M 1/36

(54) **KAMMER FÜR EIN BLUTSCHLAUCHSYSTEMS DES EXTRAKORPOALEN BLUTKREISLAUFS EINER DIALYSMASCHINE, VERWENDUNG DER KAMMER, BLUTSCHLAUCHSYSTEM SOWIE BLUTBEHANDLUNGSSYSTEM**
CHAMBER FOR A BLOOD CIRCUIT SYSTEM OF AN EXTRACORPOREAL BLOOD CIRCUIT OF A DIALYSIS MACHINE, USE OF THE CHAMBER, BLOOD TUBE SYSTEM AND BLOOD TREATMENT SYSTEM
CHAMBRE POUR UN CIRCUIT DE TRAITEMENT DE SANG D'UN CIRCUIT DE SANG EXTRACORPOREL D'UNE MACHINE DE DIALYSE , UTILISATION DE LA CHAMBRE, SYSTÈME DE FLEXIBLE POUR SANG ET SYSTÈME DE TRAITEMENT DE SANG

(30) Priorität: 21.12.2010 EP 10015895; 21.12.2010 US 201061425409 P
(43) Veröffentlichungstag der Anmeldung: 30.10.2013
(73) Patentinhaber: Fresenius Medical Care Deutschland GmbH, 61352 Bad Homburg (DE)
(72) Erfinder: REITER, Reinhold, 26013 Crema (CR) (IT); STABILINI, Paolo, 26014 Romanengo (IT)
(74) Vertreter: Herrmann, Uwe
(86) Internationale Anmeldenummer: PCT/EP2011/006450
(87) Internationale Veröffentlichungsnummer: WO 2012/084208

(56) Entgegenhaltungen:
- EP-A1- 0 062 913
- EP-A1- 2 186 533
- EP-A2- 1 210 956
- DE-A1- 3 420 278
- DE-U1- 20 011 287
- US-A- 4 765 959

## Beschreibung

Die vorliegende Erfindung, so als begrenzt bei den Ansprüchen, betrifft eine Kammer für das Blutschlauchsystem des extrakorporalen Blutkreislaufs einer Dialysemaschine, wobei die Kammer das Funktionsäquivalent einer Tropfkammer bildet, mit wenigstens einem durch eine Kammerwandung begrenzten Raum sowie mit wenigstens einem Bluteinlass und mit wenigstens einem Einlass für eine weitere Flüssigkeit, die mit dem Raum in Verbindung stehen.

Aus dem Stand der Technik ist Dokument EP0062913 bekannt. Mehr allgemain ist es bekannt, stromabwärts des Dialysators eines Dialysegerätes eine venöse Tropfkammer vorzusehen, die Bestandteil des extrakorporalen Blutkreislaufes ist. Diese venöse Tropfkammer hat die Aufgabe, eine möglichst blasenfreie Reinfusion des im Dialysator gereinigten Patientenblutes zum Patienten sicherzustellen.

Aus dem Stand der Technik ist es des weiteren bekannt, in eine solche Tropfkammer eine Infusionslösung, wie beispielsweise gereinigtes Dialysat oder eine Salzlösung einzuführen. Eine solche Zufuhr kann im Rahmen der sogenannten Predilution vor dem Dialysator und/oder im Rahmen der sogenannten Postdilution nach dem Dialysator erfolgen.

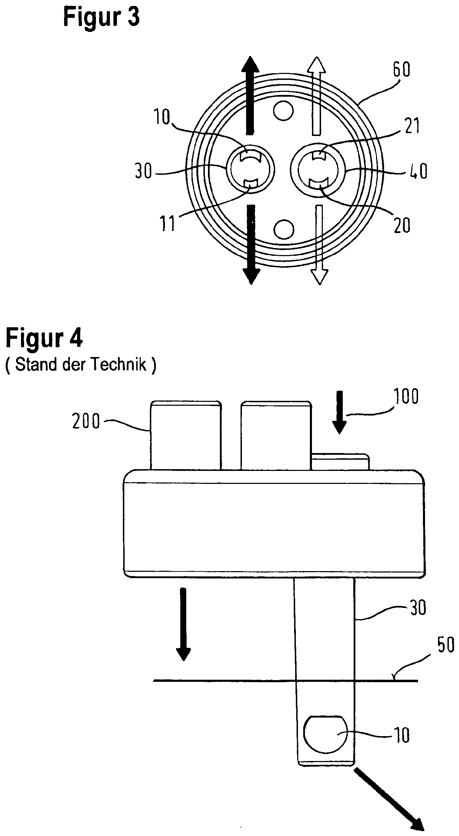

Figur 4 zeigt eine aus dem Stand der Technik bekannte Ausführungsform in Form eines Deckels oder Kopfstückes einer solchen venösen Tropfkammer, an dem ein Zuführport 100 für das im Dialysator gereinigte Blut angeordnet ist.

Mit dem Bezugszeichen 200 ist ein Zuführport für die genannte Infusionslösung gekennzeichnet.

Wie dies weiter aus Figur 4 hervorgeht, ist es bekannt, das Blut mittels eines Rohrstückes 30 in die Kammer einzuleiten, wobei das Rohrstück 30 einen Einlass 10 aufweist, durch den das Blut in die Kammer eingeführt wird, wie dies durch den Pfeil in Figur 4 gekennzeichnet ist.

Die genannte Infusionslösung wird mittels des Zuführportes 200 eingeführt, wobei der Einlaß für die Infusionslösung oberhalb des mit dem Bezugszeichen 50 gekennzeichneten Flüssigkeits- bzw. Blutpegels in der Kammer liegt. Die Infusionsflüssigkeit tropft oder strömt, wie durch den Pfeil in Figur 4 markiert, auf die Oberfläche des Blutes bzw. der Mischung aus Blut und der Infusionslösung.

Eine solche Ausgestaltung einer Tropfkammer hat den Nachteil, dass es aufgrund des Auftreffens der Infusionslösung auf die Oberfläche der in der Kammer befindlichen Flüssigkeit bzw. des Blutes zu der Bildung von Mikroblasen kommen kann, was die Auslösung eines Alarmzustandes und ggf. die Einstellung der Behandlung zur Folge haben kann.

Der vorliegenden Erfindung liegt somit die Aufgabe zugrunde, eine Kammer der eingangs genannten Art dahingehend weiterzubilden, dass die Bildung von Mikroblasen in der Tropfkammer weitgehend oder vollständig verhindert wird.

Diese Aufgabe wird durch eine Kammer mit den Merkmalen des Anspruchs 1 gelöst.

Danach ist vorgesehen, dass sowohl der wenigstens eine Bluteinlass als auch der wenigstens eine Einlass für die weitere Flüssigkeit, wie beispielsweise eine Infusionslösung, an Rohrstücken ausgebildet sind, die in Raum der Kammer hineinragen. Abweichend von der Ausgestaltung gemäß Figur 4 nach dem Stand der Technik ist somit vorgesehen, dass auch der Einlass für die Infusionsflüssigkeit bzw. für die weitere Flüssigkeit an einem Rohrstück ausgebildet ist, der in den Raum der Kammer hineinragt, in dem sich das Blut bzw. die Mischung aus Blut und der Infusionslösung befindet.

Erfindungsgemäß ist weiter vorgesehen, dass der Raum der Kammer zumindest teilweise mit Blut oder mit einer Mischung aus Blut und einer weiteren Flüssigkeit gefüllt ist oder mit einer sonstigen Flüssigkeit gefüllt ist und dass wenigstens eines der Rohrstücke eine Länge derart aufweist, dass sich der Bluteinlass und/oder der Einlass für die weitere Flüssigkeit unterhalb des Blut- bzw. Flüssigkeitspegels in der Kammer befinden. Auf diese Weise ist sichergestellt, dass zumindest im Betriebszustand der Kammer keinerlei Auftropfen, auch nicht aus geringer Höhe auf die Oberfläche des in der Kammer befindlichen Blutes bzw. auf die Oberfläche der in der Kammer befindlichen Mischung aus Blut und Infusionslösung oder sonstigen Flüssigkeit auftritt. Vielmehr wird nach einer bevorzugten Ausgestaltung der Erfindung das Blut sowie auch die weitere Flüssigkeit unterhalb des Pegels des Blutes bzw. der in der Kammer befindlichen Flüssigkeit eingeleitet. Die vorliegende Erfindung betrifft somit in dieser Ausgestaltung eine Kammer für ein Blutbehandlungssystem, deren Raum zumindest teilweise mit Blut oder mit einer Mischung aus Blut und einer weiteren Flüssigkeit oder mit einer sonstigen Flüssigkeit gefüllt ist, wobei wenigstens eines der Rohrstücke eine Länge derart aufweist, dass sich der Bluteinlass und/oder der Einlass für die weitere Flüssigkeit unterhalb des Flüssigkeitspegels bzw. des Blutpegels in dem Raum der Kammer befinden.

Dadurch ist es möglich, ein Auftropfen der weiteren Flüssigkeit auf die Oberfläche der bereits in der Kammer befindlichen Flüssigkeit bzw. Blut zu vermeiden und damit die Wahrscheinlichkeit für die Bildung von Mikroblasen zu verringern.

Der Begriff "Rohrstück ist weit auszulegen und umfasst starre wie auch elastische Elemente, die geeignet sind, Blut bzw. die weitere Flüssigkeit in die Kammer zu leiten. In Betracht kommen somit beispielsweise feste, z. B. aus Kunststoff bestehende Rohrabschnitte, Schläuche, etc.

Der Durchmesser sowie die Dimensionierung und die Form dieser Rohrstücke ist weitgehend beliebig. So kann deren Durchmesser innen- und außenseitig beispielsweise kreisförmig sein. Jedoch sind auch davon abweichende Ausgestaltungen von der Erfindung mit umfasst. Des weiteren ist die Erfindung nicht auf das Vorliegen von genau zwei Rohrstücken beschränkt. Es können auch mehr als zwei Rohrstücke vorgesehen sein, so dass das Blut beispielsweise durch mehr als ein Rohrstück und/oder die weitere Flüssigkeit durch mehr als ein Rohrstück in die Kammer eingeleitet wird.

Denkbar ist es, dass wenigstens eines der Rohrstücke derart angeordnet ist, dass es im Betriebszustand der Kammer vertikal oder in einem spitzen Winkel zur Vertikalen verläuft. Denkbar ist es somit, dass zwei oder mehr Rohrstücke vorgesehen sind, die im wesentlichen vertikal angeordnet sind und von denen eines einen oder mehrere Einlässe für das Blut und/oder das andere einen oder mehrere Einlässe für die weitere Flüssigkeit aufweist.

Die beiden Rohrstücke können parallel oder im Wesentlichen parallel zueinander verlaufen. Jedoch ist auch eine winklige Anordnung der Rohrstücke relativ zueinander denkbar.

In weiterer Ausgestaltung der Erfindung ist vorgesehen, dass der Raum der Kammer im Betriebszustand der Kammer teilweise mit Blut oder mit einer Mischung aus Blut und der wenigstens einen Flüssigkeit oder einer sonstigen Flüssigkeit gefüllt ist und dass sich oberhalb des Blut- bzw. Flüssigkeitspegels ein Luftpolster befindet.

Weiterhin kann vorgesehen sein, dass die Rohrstücke eine unterschiedliche oder dieselbe Länge aufweisen.

Denkbar ist es beispielsweise, das Rohrstück, das den oder die Einlässe für die weitere Flüssigkeit trägt genauso lang oder kürzer oder länger auszugestalten als das Rohrstück, das den oder die Bluteinlässe trägt.

Besonders vorteilhaft ist es, wenn die Länge beider Rohrstücke so ausgeführt ist, dass die Einlässe für das Blut und die weitere Flüssigkeit unterhalb des Flüssigkeitspegels in der Kammer liegen.

In weiterer Ausgestaltung der Erfindung weist wenigstens eines der Rohrstücke einen oder mehrere Bluteinlässe oder einen oder mehrere Einlässe für die weitere Flüssigkeit auf. Die Erfindung ist somit nicht darauf beschränkt, dass jedes Rohrstück nur genau einen Einlass aufweist, sondern umfasst auch den Fall, dass zwei oder mehr als zwei Einlässe pro Rohrstück vorgesehen sind.

In weiterer Ausgestaltung der Erfindung ist vorgesehen, dass der Bluteinlass und/oder der Einlass für die weitere Flüssigkeit in dem Endbereich des Rohrstückes angeordnet ist, der in die Kammer hineinragt.

Weiterhin kann vorgesehen sein, dass wenigstens eines der Rohrstücke eine Mantelfläche und eine in den Raum der Kammer ragende Endfläche aufweist und dass der wenigstens eine Bluteinlass und/oder der wenigstens eine Einlass für die weitere Flüssigkeit zumindest teilweise oder auch vollständig in der Mantelfläche angeordnet ist. Durch diese Ausgestaltung der Erfindung ist somit eine laterale, das heißt seitliche Einleitung des Blutes bzw. der weiteren Flüssigkeit denkbar. In dieser Ausgestaltung wird somit das Blut und/oder die weitere Flüssigkeit nicht in einer Richtung eingeleitet, die mit der Längsachse des Rohrstückes zusammenfällt, sondern in einer Richtung, die in einem Winkel dazu verläuft. Denkbar ist, es dass dieser Winkel im Bereich zwischen 30 ° und 150 °, vorzugsweise in einem Bereich zwischen 60° und 120° und besonders bevorzugt in einem Bereich zwischen 80° und 100° relativ zur Längsachse des Rohrstückes liegt. Liegt der Winkel beispielsweise bei ca. 90° strömt somit das Blut zunächst durch den Innenraum des Rohrstückes und dann im rechten Winkel dazu aus dem Rohrstück durch den Einlass in die Kammer.

In weiterer Ausgestaltung der Erfindung ist vorgesehen, dass wenigstens eines der Rohrstücke mehrere Bluteinlässe oder mehrere Einlässe für die weitere Flüssigkeit aufweist. Diese mehreren Einlässe können so angeordnet sein, dass das Blut in unterschiedlichen Richtungen aus dem Rohrstück in die Kammer einströmt. Entsprechendes kann für die genannte weitere Flüssigkeit gelten. Denkbar ist es, dass die beiden Einlässe so angeordnet sind, dass das Blut bzw. die weitere Flüssigkeit auf gegenüberliegenden Seiten aus den Rohrstücken ausströmt, so dass sich entgegengesetzte Strömungsrichtungen ergeben.

Denkbar ist es weiterhin, dass die mehreren Einlässe auf gleicher Höhe, das heißt im Falle vertikaler Rohrstücke auf gleicher vertikaler Position der Rohrstücke angeordnet sind und/oder dass die mehreren Einlässe eine identische Größe oder auch unterschiedliche Größen aufweisen.

Weiterhin kann vorgesehen sein, dass wenigstens eines der Rohrstücke in seinem in den Raum der Kammer ragenden Endbereich eine Wandung aufweist und dass sich der wenigstens eine Einlass angrenzend an diese Wandung befindet. Diese Wandung kann somit das in den Raum ragende Ende des Rohrstückes bilden. Angrenzend an diese Wandung können sich ein oder mehrere Einlässe für das Blut bzw. für die weitere Flüssigkeit befinden.

Alternativ zu dem oben beschriebenen Ausführungsbeispiel mit zwei Einlässen pro Rohrstück ist es selbstverständlich auch denkbar, mehr als zwei Einlässe, beispielsweise drei oder vier Einlässe pro Rohrstück auszubilden.

Dabei können diese Einlässe gleichmäßig in Umfangsrichtung über das jeweilige Rohrstück beabstandet sein. Auch eine ungleichmäßige Verteilung ist denkbar.

Der genannte Einlass des Blutes bzw. der weiteren Flüssigkeit mittels mehrerer, voneinander in Umfangsrichtung beabstandeter Einlässe bringt den Vorteil mit sich, dass einer Wirbelbildung innerhalb der Kammer wirksam entgegengewirkt wird, was wiederum den Vorteil mit sich bringt, dass das Auftreten von Mikrobläschen weitgehend oder vollständig verhindert wird.

Der oder die Einlässe für das Blut bzw. der oder die Einlässe für die weitere Flüssigkeit können so angeordnet sein, dass das Blut bzw. die weitere Flüssigkeit in radialer Richtung der Kammer bzw. auf die Kammerwandung zugerichtet, oder auch in tangentialer Richtung der Kammer bzw. in Umfangsrichtung oder auch in einer Richtung zwischen diesen beiden Richtungen eingeleitet wird.

Weiterhin kann vorgesehen sein, dass die Rohrstücke voneinander beabstandet sind, so dass also separate Einlässe für Blut einerseits und für die weitere Flüssigkeit andererseits vorliegen.

Des weiteren kann vorgesehen sein, dass die Kammer einen Grundkörper und einen den Grundkörper verschließenden Deckel oder Kopfstück aufweist und dass die Rohrstücke an dem Deckel bzw. Kopfstück angeordnet sind und sich vorzugsweise durch den Deckel bzw. das Kopfstück hindurch erstrecken. Der Deckel bzw. das Kopfstück kann fest mit dem Grundkörper in Verbindung stehen oder auch lösbar von dem Grundkörper ausgeführt sein. Im Falle einer festen Ausgestaltung ist es denkbar, Grundkörper und Deckel bzw. Kopfstück einteilig auszuführen.

Die vorliegende Erfindung betrifft des weiteren die Verwendung einer Kammer nach einem der Ansprüche 1 bis 15 in einem Blutbehandlungssystem. Vorzugsweise wird die Kammer als venöse Kammer verwendet, die stromabwärts eines Dialysators angeordnet ist.

Der Raum der Kammer kann im Betriebszustand der Kammer zumindest teilweise mit Blut oder mit einer Mischung aus Blut und der weiteren Flüssigkeit oder einer sonstigen Flüssigkeit gefüllt sein und wenigstens eines der Rohrstücke kann eine Länge derart aufweisen, dass sich der Bluteinlass und/oder der Einlass für die weitere Flüssigkeit unterhalb des Blut- bzw. Flüssigkeitspegels in der Kammer befindet. Wie bereits oben ausgeführt, lässt sich damit die besonders bevorzugte Ausgestaltung der Erfindung realisieren, dass ein Auftropfen sowohl des Blutes auch der weiteren Flüssigkeit auf die Oberfläche des in der Kammer befindlichen Blutes bzw. die Oberfläche der in der Kammer befindlichen Flüssigkeit verhindert wird.

In weiterer Ausgestaltung der Erfindung ist vorgesehen, dass der Raum der Kammer im Betriebszustand der Kammer teilweise mit Blut oder mit einer Mischung aus Blut und der weiteren Flüssigkeit oder einer sonstigen Flüssigkeit gefüllt ist und dass sich oberhalb des Blutes ein Luftpolster befindet.

Weiterhin kann vorgesehen sein, dass im Betriebszustand der Kammer wenigstens eines der Rohrstücke vertikal oder in einem spitzen Winkel zur Vertikalen verläuft.

Wie bereits oben ausgeführt, ist es vorteilhaft, wenn der oder die Einlässe für das Blut und/oder für die weitere Flüssigkeit derart an dem Rohrstück ausgebildet sind, dass das Blut und/oder die weitere Flüssigkeit in einem Winkel zur Längsachse des Rohrstückes aus dem Rohrstück austritt. Dieser Winkel kann beispielsweise im Bereich zwischen 30 ° und 150 °, vorzugsweise in einem Bereich zwischen 60° und 120° und besonders bevorzugt in einem Bereich zwischen 80° und 100° liegen.

Wenigstens eines der Rohrstücke kann mehrere Einlässe aufweisen, die derart an dem Rohrstück angeordnet sind, dass das Blut und/oder die weitere Flüssigkeit in wenigstens zwei unterschiedlichen Richtungen aus dem jeweiligen Rohrstück abgegeben wird. Auch dadurch kann die Bildung von Wirbeln in der Kammer und damit auch das Auftreten von Mikroblasen weitgehend oder vollständig verhindert werden.

Bei der weiteren Flüssigkeit handelt es sich um eine Infusionslösung, insbesondere um eine Dialyselösung oder um eine Salzlösung, wie beispielsweise eine Kochsalzlösung. Diese wird in dem Raum der Kammer mit Blut gemischt und dann diese Mischung dem Patienten zugeführt.

Die vorliegende Erfindung betrifft des weiteren ein Blutschlauchssystem mit wenigstens einer Kammer nach einem der Ansprüche 1 bis 15. Dabei kann vorgesehen sein, dass das Blutschlauchsystem über einen Predilutions-Anschluss und/oder über einen Postdilutions-Anschluss verfügt und dass einer oder beide dieser Anschlüsse mit dem Rohrstück für die weitere Flüssigkeit in Fluidverbindung stehen oder zur Ausbildung einer Fluidverbindung verbindbar sind.

Die vorliegende Erfindung betrifft des weiteren ein Blutbehandlungssystem mit wenigstens einer Kammer nach einem der Ansprüche 1 bis 15 oder mit wenigstens einem Blutschlauchssystem nach Anspruch 24 oder 25.

In vorteilhafter Ausgestaltung der Erfindung ist vorgesehen, dass es sich bei dem Blutbehandlungssystem um eine Dialysemaschine handelt. Die Dialysemaschine kann ausgeführt sein, um mit dieser eine Hämodialyse, eine Hämofiltration oder auch eine Hämodiafiltration durchzuführen.

Weiterhin kann vorgesehen sein, dass die weitere Flüssigkeit durch die Dialysemaschine bereitgestellt wird.

Weitere Einzelheiten und Vorteile der Erfindung werden anhand eines in der Zeichnung dargestellten Ausführungsbeispiels näher erläutert. Es zeigen:
- Figur 1:: eine perspektivische Darstellung eines Deckels einer erfindungsgemäßen Kammer in einer Ansicht von schräg unten,
- Figur 2:: eine Schnittdarstellung durch ein Rohrstück mit Einlässen für Blut oder die weitere Flüssigkeit,
- Figur 3:: eine schematische Draufsicht auf die Anordnung gemäß Figur 1 mit angedeuteten Strömungsrichtungen für das Blut sowie für die weitere Flüssigkeit und
- Figur 4:: eine Seitenansicht eines Deckels einer venösen Tropfkammer aus dem Stand der Technik.

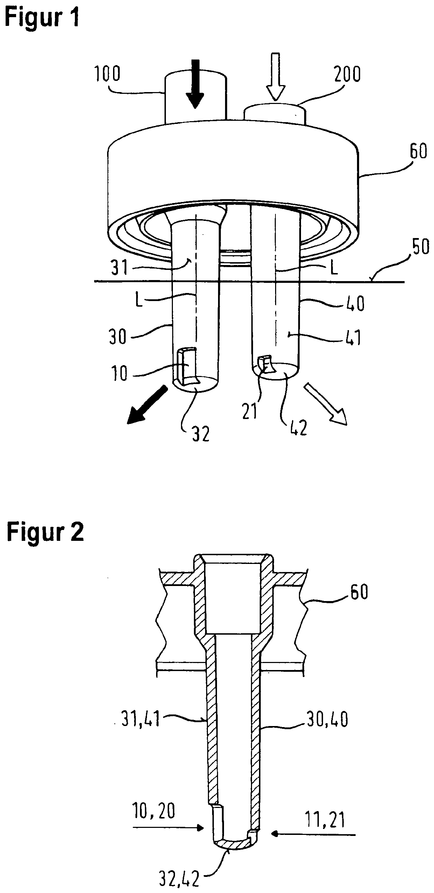

Figur 1 zeigt in perspektivischer Ansicht von schräg unten einen Kopf- bzw. Deckelbereich 60 einer venösen Kammer eines extrakorporalen Blutkreislaufes eines Dialysegerätes.

Mit dem Bezugszeichen 100 ist der Zuführport für das Blut und mit dem Bezugszeichen 200 der Zuführport für die Infusionslösung gekennzeichnet. Der Zuführport 100 steht mit dem extrakorporalen Blutkreislauf in Verbindung. Durch den Zuführport 100 gelangt das in dem Dialysator gereinigte Blut in die erfindungsgemäße venöse Kammer, deren Deckel bzw. Kopfstück 60 in Figur 1 gezeigt ist.

Der Zuführport 200 für die Infusionslösung steht mit einer geeigneten Quelle für die Infusionslösung, wie beispielsweise Dialysat, einer Kochsalzlösung, etc. in Verbindung. Durch diesen Port 200 wird die Infusionslösung der Kammer zugeführt. Diese Zufuhr findet in dem dargestellten Ausführungsbeispiel stromabwärts des Dialysators statt, so dass eine Postdilution vorliegt.

Grundsätzlich ist die Erfindung jedoch nicht darauf beschränkt, sondern ermöglicht den Einsatz einer Kammer auch im Bereich der Predilution.

In Figur 1 ist mit dem Bezugszeichen 50 der Blutpegel bzw. der Pegel der in der Kammer befindlichen Flüssigkeit gekennzeichnet. Diese Flüssigkeit besteht entweder nur aus Blut oder aus einer Mischung aus Blut und der genannten Infusionsflüssigkeit oder aus einer sonstigen Flüssigkeit. Wie dies weiter aus Figur 1 hervorgeht, steht der Zuführport 100 mit dem Rohrstück 30 vorzugsweise fest bzw. einteilig in Verbindung und der Zuführport 200 vorzugsweise fest bzw. einteilig mit dem dazu parallel verlaufenden Rohrstück 40. Beide Rohrstücke 30, 40 sind voneinander beabstandet, wie dies aus Figur 1 hervorgeht. Die Zuführports 100, 200 können integraler Bestandteil der Rohrstücke 30, 40 sein oder mit diesen in geeigneter Weise in Verbindung stehen. Auch ist es denkbar, dass die Rohrstücke 30, 40 und/oder die Zuführports 100, 200 integrale Bestandteile des Deckels bzw. des Kopfteils 60 der Kammer sind oder mit diesen anderweitig in Verbindung stehen, beispielsweise in diesen eingesteckt sind.

Mit dem Bezugszeichen L sind die Längsachsen der beiden Rohrstücke 30, 40 gekennzeichnet, die in den Raum der Kammer hineinragen, die kopfseitig durch das Kopfstück 60 bzw. durch den Deckel 60 abgeschlossen wird.

Wie dies weiter aus Figur 1 hervorgeht, weist jedes der Rohrstücke 30, 40 in seiner Mantelfläche 31, 41 einen Einlass 10 bzw. 21 auf, durch den einerseits Blut und andererseits die Infusionsflüssigkeit in die bereits in der Kammer befindliche Flüssigkeit bzw. das Blut gelangt.

Aus Figur 1 ergibt sich weiter, dass sowohl der Einlass 10 für das Blut als auch der Einlass 21 für die weitere Lösung bzw. für die Infusionsflüssigkeit unterhalb des Pegels 50 der bereits in der Kammer befindlichen Flüssigkeit bzw. des Blutes liegt. Aus Figur 1 ergibt sich weiter, dass die Einlässe 10, 21 nicht im nach unten weisenden Endbereich 32, 42 der Rohrstücke 30, 40, sondern zumindest auch oder ausschließlich lateral angeordnet sind, so dass das Blut bzw. die weitere Flüssigkeit seitlich oder zumindest auch in einer seitlichen Richtung aus den Rohrstücken 30, 40 austritt.

Gemäß dem vorliegenden Ausführungsbeispiel ist somit vorgesehen, dass sowohl der Einlass für die Infusionslösung als auch der Einlass für das Blut unterhalb des Pegels 50 des bereits in der Kammer befindlichen Blutes bzw. der Mischung aus Blut und Infusionslösung oder einer sonstigen Flüssigkeit liegt.

Des weiteren ist gemäß dem vorliegenden Ausführungsbeispiel vorgesehen, dass separate Einlässe für Blut einerseits und für die weitere Lösung andererseits vorliegen.

Wie dies des weiteren aus Figur 1 entnehmbar ist, ist das Rohrstück 40, das den Einlass für die weitere Lösung trägt, etwas kürzer ausgebildet, als das Rohrstück 30, das den Einlass 10 für das Blut trägt. Grundsätzlich ist es auch denkbar, diese beiden Rohrstücke 30, 40 identisch auszuführen bzw. die Höhe bzw. Position der jeweiligen Einlässe 10, 21 auf gleicher Höhe vorzunehmen oder das Rohrstück 40 länger auszuführen als das Rohrstück 30.

Figur 2 zeigt eine Schnittdarstellung durch das Rohrstück 30 oder auch durch das Rohrstück 40. Wie dies aus Figur 2 hervorgeht, ist das Rohrstück 30, 40 integraler Bestandteil des Deckels und vorzugsweise einstückig mit diesem ausgebildet. Durch die vorliegende Erfindung ist somit ein integrierter Infusionsport realisierbar.

Aus Figur 2 ergibt sich des weiteren, dass sowohl das Rohrstück 30 zur Zufuhr von Blut als auch das Rohrstück 40 zur Zufuhr der weiteren Flüssigkeit mit jeweils zwei Einlässen 10, 11; 20, 21 ausgebildet ist die unmittelbar an die endseitige Wandung 32, 42 angrenzen. Wie dies auch aus Figur 3 hervorgeht, sind diese Einlässe jeweils gegenüberliegend angeordnet, was zur Folge hat, dass das Blut bzw. die weitere Lösung an gegenüberliegenden Seiten aus dem jeweiligen Rohrstück 30, 40 austritt und in die bereits in der Kammer befindliche Flüssigkeit eintritt. Dies ergibt sich beispielsweise aus Figur 3. Hier ist mit dem Bezugszeichen 30 das Rohrstück zur Zufuhr von Blut und mit dem Bezugszeichen 10, 11 die Einlässe für Blut gekennzeichnet. Diese Einlässe sind an dem Rohrstück 30 seitlich, das heißt lateral und gegenüberliegend angeordnet, was zur Folge hat, dass wie in Figur 3 dargestellt, das Blut in gegenüberliegenden Richtungen, das heißt in entgegengesetzten Richtungen aus dem Rohrstück 30 abgegeben wird.

Dies gilt für die Ausbildung des Rohrstückes 40 zur Zufuhr der weiteren Flüssigkeit entsprechend. Auch hier sind zwei gegenüberliegende Einlässe 20, 21 vorgesehen, die ebenfalls eine unterschiedliche Größe aufweisen. Wie in Figur 3 ersichtlich, sind die Strömungsrichtungen des Blutes sowie der weiteren Flüssigkeit beim Ausströmen aus den Rohrstücken 30 und 40 parallel. Von der Erfindung ist jedoch auch eine nicht parallele Ausströmung von Blut einerseits und der weiteren Lösung andererseits umfasst.

Grundsätzlich ist von der Erfindung auch der Fall umfasst, dass die jeweiligen Einlässe 10, 11 bzw. 20, 21 mit einer identischen Größe ausgeführt sind. Die Einlässe 10, 11 bzw. 20, 21 können an dem Rohrstück auf identischer Höhe oder auch auf unterschiedlichen Höhen angeordnet sein.

Wie dies weiter aus Figur 3 hervorgeht, sind die Einlässe in dem hier dargestellten Ausführungsbeispiel so angeordnet, dass die Einströmrichtung des Blutes bzw. der weiteren Flüssigkeit weder radial relativ zur zylindrischen bzw. im Querschnitt kreisförmigen Mantelfläche des Gehäuses bzw. des Deckels der Kammer erfolgt, noch tangential. Vielmehr ist hier vorgesehen, dass die Einströmrichtung des Blutes bzw. der weiteren Flüssigkeit in eine Richtung erfolgt, die zwischen der radialen und der tangentialen Richtung liegt. Durch diese Ausgestaltung der Erfindung wird eine bessere Durchmischung des Blutes und der Infusionsflüssigkeit erreicht und es gibt keinen Wirbeleffekt, der dazu führen würde, dass sich der Pegel in der Mitte der Kammer absenkt und an deren Seiten ansteigt. Vielmehr wird durch die dargestellte Ausführungsform erreicht, dass der Fluidpegel 50 bzw. die Oberfläche der Flüssigkeit in der Kammer weitgehend eben bleibt, so dass die Wirbelbildung weitgehend oder vollständig verhindert wird, was wiederum zur Folge hat, dass das Auftreten von Mikrobläschen oder Schaum verhindert wird.

Die in den Figuren 1 bis 3 dargestellte venöse Tropfkammer mit getrennten Einlässen für Blut und die weitere Flüssigkeit sowie mit der speziellen Ausgestaltung der Einlässe für Blut und Flüssigkeit am Rohrstück bringt den Vorteil mit sich, dass weder ein Auftropfen von Blut und/oder der weiteren Lösung auf die Oberfläche der in der Kammer befindlichen Flüssigkeit bzw. des Blutes erfolgt sowie den weiteren Vorteil, dass eine Wirbelbildung und damit eine Schaumbildung oder eine Mikrobläschenbildung weitgehend oder vollständig verhindert wird. Der oder die Rohrstücke 30, 40 können durch das Verfahren des Injection Moulding hergestellt werden. Das Vorsehen der weiteren Öffnungen 11, 21 bringt bei diesem Verfahren hinsichtlich der Stabilität und der Einfachheit der Durchführung des Verfahrens Vorteile. Vorzugsweise ist also vorgesehen, dass der Deckel bzw. das Kopfstück 60 insgesamt oder wenigstens die Rohrstücke 30, 40 durch das Verfahren das Injection Moulding hergestellt werden.

Die erfindungsgemäße Kammer ist insbesondere vorteilhaft anwendbar im Rahmen der Hämodiafiltration-Postdilutions-Behandlung und vorzugsweise im Rahmen der Online-HDF-Postdilution.

Bei dem Zuführport 200 handelt es sich somit vorzugsweise um einen Anschluß für eine Infusionslösung, die dem Blut nach dessen Reinigung im Dialysator zugeführt wird.

## Patentansprüche

1. Kammer für das Blutschlauchsystem des extrakorporalen Blutkreislaufs einer Dialysemaschine, wobei die Kammer das Funktionsäquivalent einer Tropfkammer bildet, mit wenigstens einem durch eine Kammerwandung begrenzten Raum sowie mit wenigstens einem Bluteinlass (10, 11) und mit wenigstens einem Einlass (20, 21) für eine Infusionslösung, die mit dem Raum in Verbindung stehen,
**dadurch gekennzeichnet,**
**dass** sowohl der wenigstens eine Bluteinlass (10, 11) als auch der wenigstens eine Einlass (20, 21) für die Infusionslösung an Rohrstücken (30, 40) ausgebildet sind, die in den Raum der Kammer hineinragen, wobei der Raum der Kammer im Betriebszustand der Kammer zumindest teilweise mit Blut oder mit einer Mischung aus Blut und der Infusionslösung oder einer sonstigen Flüssigkeit gefüllt ist und wobei beide der Rohrstücke (30, 40) eine Länge derart aufweisen, dass sich der Bluteinlass (10, 11) und der Einlass (20, 21) für die Infusionslösung unterhalb des Blut- bzw. Flüssigkeitspegels (50) in der Kammer befinden.

2. Kammer nach Anspruch 1, **dadurch gekennzeichnet, dass** wenigstens eines der Rohrstücke (30, 40) derart angeordnet ist, dass es im Betriebszustand der Kammer vertikal oder in einem spitzen Winkel zur Vertikalen verläuft; und/oder dass die Rohrstücke (30, 40) parallel oder im Wesentlichen parallel zueinander verlaufen; und/oder dass die Rohrstücke (30, 40) eine unterschiedliche oder dieselbe Länge aufweisen.

3. Kammer nach einem der vorhergehenden Ansprüche, **dadurch gekennzeichnet, dass** der Raum der Kammer im Betriebszustand der Kammer teilweise mit Blut oder mit einer Mischung aus Blut und der Infusionslösung oder einer sonstigen Flüssigkeit gefüllt ist und dass sich oberhalb des Blut- bzw. Flüssigkeitspegels (50) ein Luftpolster befindet.

4. Kammer nach einem der vorhergehenden Ansprüche, **dadurch gekennzeichnet, dass** wenigstens eines der Rohrstücke (30, 40) einen oder mehrere Bluteinlässe (10, 11) oder einen oder mehrere Einlässe (20, 21) für die Infusionslösung aufweist; und/oder dass der Bluteinlass (10, 11) und/oder der Einlass (20, 21) für die Infusionslösung in dem Endbereich des Rohrstückes (30, 40) angeordnet ist, der in die Kammer hineinragt.

5. Kammer nach einem der vorhergehenden Ansprüche, **dadurch gekennzeichnet, dass** wenigstens eines der Rohrstücke (30, 40) eine Mantelfläche (31, 41) und eine in den Raum der Kammer ragende Endfläche (32, 42) aufweist und dass der wenigstens eine Bluteinlass (10, 11) und/oder der wenigstens eine Einlass (20, 21) für die Infusionslösung zumindest teilweise in der Mantelfläche (31, 41) angeordnet ist.

6. Kammer nach einem der vorhergehenden Ansprüche, **dadurch gekennzeichnet, dass** der Bluteinlass (10, 11) und/oder der Einlass (20, 21) für die Infusionslösung derart in dem Rohrstück (30, 40) ausgebildet ist, dass das Blut und/oder die Infusionslösung in einem Winkel zur Längsachse (L) des Rohrstückes (30, 40) aus dem Rohrstück (30, 40) austritt, wobei der Winkel vorzugsweise im Bereich zwischen 30° und 150°, weiter vorzugsweise in einem Bereich zwischen 60° und 120° und besonders bevorzugt in einem Bereich zwischen 80° und 100° liegt.

7. Kammer nach einem der vorhergehenden Ansprüche, **dadurch gekennzeichnet, dass** wenigstens eines vorzugsweise beider oder mehrere der Rohrstücke (30, 40) mehrere Bluteinlässe (10, 11) oder mehrere Einlässe (20, 21) für die Infusionslösung aufweisen.

8. Kammer nach einem der vorhergehenden Ansprüche, **dadurch gekennzeichnet, dass** die mehreren Einlässe derart an dem Rohrstück (30, 40) angeordnet sind, dass das Blut und/oder die Infusionslösung im Betriebszustand der Kammer in wenigstens zwei unterschiedlichen Richtungen aus dem jeweiligen Rohrstück (30, 40) abgegeben wird.

9. Kammer nach Anspruch 7, **dadurch gekennzeichnet, dass** die mehreren Einlässe (10, 11; 20, 21) an gegenüberliegenden Seiten des jeweiligen Rohrstückes (30, 40) angeordnet sind und/oder das die mehreren Einlässe (10, 11; 20, 21) auf gleicher Höhe des jeweiligen Rohrstückes (30, 40) angeordnet sind und/oder dass die mehreren Einlässe (10, 11; 20, 21) eine identische Größe oder unterschiedliche Größen aufweisen.

10. Kammer nach einem der vorhergehenden Ansprüche, **dadurch gekennzeichnet, dass** wenigstens eines der Rohrstücke (30, 40) in seinem in den Raum der Kammer ragenden Endbereich eine Wandung (32, 42) aufweist und dass sich der wenigstens eine Einlass (10, 11; 20, 21) angrenzend an diese Wandung (32, 42) befindet.

11. Kammer nach einem der vorhergehenden Ansprüche, **dadurch gekennzeichnet, dass** die Rohrstücke (30, 40) voneinander beabstandet sind, so dass separate Einlässe (10, 11; 20, 21) für Blut und für die Infusionslösung vorliegen.

12. Kammer nach einem der vorhergehenden Ansprüche, **dadurch gekennzeichnet, dass** die Kammer einen Grundkörper und einen den Grundkörper verschließenden Deckel oder Kopfstück (60) aufweist und dass die Rohrstücke (30, 40) an dem Deckel oder Kopfstück (60) angeordnet sind und sich vorzugsweise durch den Deckel oder das Kopfstück (60) hindurch erstrecken.

13. Verwendung einer Kammer nach einem der Ansprüche 1 bis 12 zur Herstellung eines Blutschlauchsystems für den extrakorporalen Blutkreislauf einer Dialysemaschine, wobei die Kammer insbesondere als venöse Kammer Verwendung findet.

14. Blutschlauchssystem für den extrakorporalen Blutkreislauf einer Dialysemaschine mit wenigstens einer Kammer nach einem der Ansprüche 1 bis 12, wobei das Blutschlauchsystem vorzugsweise über einen Predilutions-Anschluss und/oder über einen Postdilutions-Anschluss verfügt und dass einer oder beide dieser Anschlüsse mit dem Rohrstück (40) für die Infusionslösung in Fluidverbindung stehen oder zur Ausbildung einer Fluidverbindung verbindbar sind.

15. Dialysemaschine mit wenigstens einer Kammer nach einem der Ansprüche 1 bis 12 oder mit wenigstens einem Blutschlauchssystem nach Anspruch 14, wobei die Dialysemaschine vorzugsweise derart ausgebildet ist, dass die Infusionslösung durch die Dialysemaschine bereitgestellt wird.

## Claims

1. A chamber for a blood tube system of the extracorporeal blood circuit of a dialysis machine, wherein the chamber forms the functional equivalent of a drip chamber, with at least one space defined by a chamber wall and with at least one blood inlet (10, 11) and with at least one inlet (20, 21) for an infusion solution, which are connected with the space,
**characterized in**
**that** both the at least one blood inlet (10, 11) and the at least one inlet (20, 21) for the infusion solution are formed on tube sections (30, 40) which protrude into the space of the chamber, wherein in the operating condition of the chamber the space of the chamber is at least partly filled with blood or with a mixture of blood and the infusion solution or some other fluid and wherein both of the tube sections (30, 40) have a length such that the blood inlet (10, 11) and the inlet (20, 21) for the infusion solution are located below the blood or fluid level (50) in the chamber.

2. The chamber according to claim 1, **characterized in that** at least one of the tube sections (30, 40) is arranged such that in the operating condition of the chamber it extends vertically or at an acute angle to the vertical; and/or that the tube sections (30, 40) extend parallel or substantially parallel to each other; and/or that the tube sections (30, 40) have a different length or the same length.

3. The chamber according to any of the preceding claims, **characterized in that** in the operating condition of the chamber the space of the chamber is partly filled with blood or with a mixture of blood and the infusion solution or some other fluid and that above the blood or fluid level (50) an air cushion is disposed.

4. The chamber according to any of the preceding claims, **characterized in that** at least one of the tube sections (30, 40) includes one or more blood inlets (10, 11) or one or more inlets (20, 21) for the infusion solution; and/or that the blood inlet (10, 11) and/or the inlet (20, 21) for the infusion solution is arranged in the end region of the tube section (30, 40) which protrudes into the chamber.

5. The chamber according to any of the preceding claims, **characterized in that** at least one of the tube sections (30, 40) includes a shell surface (31, 41) and an end surface (32, 42) protruding into the space of the chamber and that the at least one blood inlet (10, 11) and/or the at least one inlet (20, 21) for the infusion solution is at least partly arranged in the shell surface (31, 41).

6. The chamber according to any of the preceding claims, **characterized in that** the blood inlet (10, 11) and/or the inlet (20, 21) for the infusion solution is formed in the tube section (30, 40) such that the blood and/or the infusion solution exits from the tube section (30, 40) at an angle to the longitudinal axis (L) of the tube section (30, 40), wherein the angle preferably lies in the range between 30° and 150°, more preferably in a range between 60° and 120°, and particularly preferably in a range between 80° and 100°.

7. The chamber according to any of the preceding claims, **characterized in that** at least one, preferably both or a plurality of the tube sections (30, 40) include a plurality of blood inlets (10, 11) or a plurality of inlets (20, 21) for the infusion solution.

8. The chamber according to any of the preceding claims, **characterized in that** the plurality of inlets are arranged on the tube section (30, 40) such that in the operating condition of the chamber the blood and/or the infusion solution is output from the respective tube section (30, 40) in at least two different directions.

9. The chamber according to claim 7, **characterized in that** the plurality of inlets (10, 11; 20, 21) are arranged on opposite sides of the respective tube section (30, 40) and/or that the plurality of inlets (10, 11; 20, 21) are arranged at the same height of the respective tube section (30, 40) and/or that the plurality of inlets (10, 11; 20, 21) have an identical size or a different size.

10. The chamber according to any of the preceding claims, **characterized in that** at least one of the tube sections (30, 40) has a wall (32, 42) in its end region protruding into the space of the chamber and that the at least one inlet (10, 11; 20, 21) is located adjacent to this wall (32, 42).

11. The chamber according to any of the preceding claims, **characterized in that** the tube sections (30, 40) are spaced from each other, so that separate inlets (10, 11; 20, 21) are present for blood and for the infusion solution.

12. The chamber according to any of the preceding claims, **characterized in that** the chamber includes a base body and a lid or headpiece (60) closing the base body and that the tube sections (30, 40) are arranged on the lid or headpiece (60) and preferably extend through the lid or headpiece (60).

13. Use of a chamber according to any of claims 1 to 12 for manufacturing a blood tube system for the extracorporeal blood circuit of a dialysis machine, wherein the chamber in particular is used as venous chamber.

14. A blood tube system for the extracorporeal blood circuit of a dialysis machine with at least one chamber according to any of claims 1 to 12, wherein the blood tube system preferably is provided with a predilution port and/or with a postdilution port and that one or both of these ports are in fluid connection with the tube section (40) for the infusion solution or are connectable to form a fluid connection.

15. A dialysis machine with at least one chamber according to any of claims 1 to 12 or with at least one blood tube system according to claim 14, wherein the dialysis machine preferably is formed such that the infusion solution is provided by the dialysis machine.

## Revendications

1. Chambre pour le système de tuyauterie sanguine pour le circuit sanguin extracorporel d'une machine de dialyse, la chambre formant l'équivalent fonctionnel d'une chambre d'égouttage, avec au moins un espace délimité par une paroi de chambre et avec au moins un orifice d'entrée de sang (10, 11) et avec au moins un orifice d'entrée (20, 21) pour une solution de perfusion, qui sont reliés à l'espace,
**caractérisée en ce que**
le au moins un orifice d'entrée de sang (10, 11) aussi bien que le au moins un orifice (20, 21) pour la solution de perfusion sont formés sur des pièces de tube (30, 40) qui font saillie dans l'espace de la chambre, l'espace de la chambre dans l'état de fonctionnement de la chambre au moins partiellement étant rempli de sang ou d'un mélange de sang and de la solution de perfusion ou d'un autre fluide quelconque, et les deux pièces de tube (30, 40) possédant une longueur telle que l'orifice d'entrée de sang (10, 11) et l'orifice d'entrée (20, 21) pour la solution de perfusion se trouvent au-dessous du niveau de sang ou de fluide (50) dans la chambre.

2. Chambre selon la revendication 1, **caractérisée en ce qu'**au moins une des pièces de tube (30, 40) est agencée de sorte que dans l'état de fonctionnement de la chambre elle s'étend verticalement ou en formant un angle aigu avec la verticale ; et/ou que les pièces de tube (30, 40) s'étendent parallèlement ou sensiblement parallèlement l'une à l'autre ; et/ou que les pièces de tube (30, 40) possèdent une longueur différente ou la même longueur.

3. Chambre selon l'une quelconque des revendications précédentes,
**caractérisée en ce que** dans l'état de fonctionnement de la chambre l'espace de la chambre est partiellement rempli de sang ou d'un mélange de sang et de la solution de perfusion ou d'un autre fluide quelconque et qu'au-dessus du niveau de sang ou de fluide (50) se trouve un coussin d'air.

4. Chambre selon l'une quelconque des revendications précédentes,
**caractérisée en ce qu'**au moins une des pièces de tube (30, 40) possède un ou plusieurs orifices d'entrée de sang (10, 11) ou un ou plusieurs orifices d'entrée (20, 21) pour la solution de perfusion ; et/ou que l'orifice d'entrée de sang (10, 11) et/ou l'orifice d'entrée (20, 21) pour la solution de perfusion est agencé dans la région d'extrémité de la pièce de tube (30, 40), qui fait saillie dans la chambre.

5. Chambre selon l'une quelconque des revendications précédentes,
**caractérisée en ce qu'**au moins une des pièces de tube (30, 40) possède une surface d'enveloppe (31, 41) et une surface d'extrémité (32, 42) faisant saillie dans l'espace de la chambre et que le au moins un orifice d'entrée de sang (10, 11) et/ou le au moins un orifice d'entrée (20, 21) pour la solution de perfusion est agencé au moins partiellement dans la surface d'enveloppe (31,41).

6. Chambre selon l'une quelconque des revendications précédentes,
**caractérisée en ce que** l'orifice d'entrée de sang (10, 11) et/ou l'orifice d'entrée (20, 21) pour la solution de perfusion est formé dans la pièce de tube (30, 40) de sorte que le sang et/ou la solution de perfusion sort de la pièce de tube (30, 40) dans un angle à l'axe longitudinal (L) de la pièce de tube (30, 40), l'angle se situant de préférence dans une gamme comprise entre 30° et 150°, plus préférablement dans une gamme comprise entre 60° et 120°, et encore plus préférablement dans une gamme comprise entre 80° et 100°.

7. Chambre selon l'une quelconque des revendications précédentes,
**caractérisée en ce qu'**au moins une, de préférence les deux ou plusieurs de pièces de tube (30, 40) possèdent plusieurs orifices d'entrée de sang (10, 11) ou plusieurs orifices d'entrée (20, 21) pour la solution de perfusion.

8. Chambre selon l'une quelconque des revendications précédentes,
**caractérisée en ce que** les plusieurs orifices d'entrée sont agencés sur la pièce de tube (30, 40) de sorte que dans l'état de fonctionnement de la chambre le sang et/ou la solution de perfusion est émis(e) depuis la pièce de tube (30, 40) respective dans au moins deux directions différentes.

9. Chambre selon la revendication 7, **caractérisée en ce que** les plusieurs orifices d'entrée (10, 11 ; 20, 21) sont agencés sur les côtés opposés de la pièce de tube (30, 40) respective et/ou que les plusieurs orifices d'entrée (10, 11 ; 20, 21) sont agencés à la même hauteur que la pièce de tube (30, 40) respective et/ou que les plusieurs orifices d'entrée (10, 11 ; 20, 21) possèdent une taille identique ou des tailles différentes.

10. Chambre selon l'une quelconque des revendications précédentes,
**caractérisée en ce qu'**au moins une des pièces de tube (30, 40) possède une paroi (32, 42) dans sa région d'extrémité faisant saillie dans l'espace de la chambre et que le au moins un orifice d'entrée (10, 11 ; 20, 21) se trouve adjacent à cette paroi (32, 42).

11. Chambre selon l'une quelconque des revendications précédentes,
**caractérisée en ce que** les pièces de tube (30, 40) sont espacées les unes des autres de sorte que des orifices d'entrée (10, 11 ; 20, 21) séparés existent pour le sang and pour la solution de perfusion.

12. Chambre selon l'une quelconque des revendications précédentes,
**caractérisée en ce que** la chambre possède un corps de base et un couvercle ou une pièce de tête fermant le corps de base, et que les pièces de tube (30, 40) sont agencées sur le couvercle ou la pièce de tête (60) et de préférence s'étendent à travers le couvercle ou la pièce de tête (60).

13. Utilisation d'une chambre selon l'une quelconque des revendications 1 à 12 pour la fabrication d'un système de tuyauterie sanguine pour le circuit sanguin extracorporel d'une machine de dialyse, la chambre étant utilisée en particulier comme chambre veineuse.

14. Système de tuyauterie sanguine pour le circuit sanguin extracorporel d'une machine de dialyse avec au moins une chambre selon l'une quelconque des revendications 1 à 12, dans lequel le système de tuyauterie sanguine de préférence possède un port de prédilution et/ou un port de postdilution et qu'un port ou les deux ports sont en communication fluidique avec la pièce de tube (40) pour la solution de perfusion ou peuvent être liés pour former une communication fluidique.

15. Machine de dialyse avec au moins une chambre selon l'une quelconque des revendications 1 à 12 ou avec au moins un système de tuyauterie sanguine selon la revendication 14, la machine de dialyse de préférence étant formée de sorte que la solution de perfusion est fournie par la machine de dialyse.
